# EUROPÄISCHE PATENTSCHRIFT

(11) **EP 2 676 698 B1**
(45) Veröffentlichungstag und Bekanntmachung des Hinweises auf die Patenterteilung: **03.09.2014**
(21) Anmeldenummer: 13168212.2
(22) Anmeldetag: 17.05.2013
(51) Int. Cl.: A61N 1/375, A61N 1/372, H01R 13/62, H01R 24/28

(54) **Adapter zum mechanischen und elektrischen Anschluss einer implantierbaren Elektrode an mindestens einen Testanschluss-Kontakt**
Adapter for mechanically and electrically connecting an implantable electrode to at least one test terminal contact
Adaptateur pour le raccordement mécanique et électrique d'une électrode pouvant être implantée sur au moins un contact de raccord de test

(30) Priorität: 18.06.2012 US 201261660838 P
(43) Veröffentlichungstag der Anmeldung: 25.12.2013
(73) Patentinhaber: BIOTRONIK SE & Co. KG, 12359 Berlin (DE)
(72) Erfinder: Stump, Joachim, 10245 Berlin (DE); Bartels, Klaus, 10115 Berlin (DE); Mieke, Marion, 14193 Berlin (DE)
(74) Vertreter: Lindner-Vogt, Karin L.

(56) Entgegenhaltungen:
- US-A1- 2002 095 079
- US-A1- 2005 177 199
- US-A1- 2006 030 918
- US-A1- 2006 258 193
- US-A1- 2008 015 668
- US-A1- 2012 130 397

## Beschreibung

Die Erfindung betrifft einen Adapter zum mechanischen und elektrischen Anschluss einer implantierbaren Elektrode mit ihrem proximalen, mindestens einen Anschluss-Kontakt für eine Elektrodenfläche der Elektrode aufweisenden Anschluss-Stecker an mindestens einen Testanschluss-Kontakt, insbesondere an eine Krokodilklemme, einer Messeinrichtung.

Zum Hintergrund der Erfindung ist darauf zu verweisen, dass beispielsweise Herzschrittmacher oder sogenannte ICDs (Intra Cardiac Devices) implantierbare Stimulationselektroden zur elektrischen Verbindung der Stimulationseinrichtung mit einem bestimmten Körpergewebe aufweisen. So umfassen implantierbare Stimulationselektroden Mess-/Schrittmacher-Elektroden, Schockelektroden, epikardiale Elektroden, endokardiale Elektroden, atriale oder ventrikuläre Elektroden, uni- oder bipolare Elektroden usw.

Solche Stimulationselektroden umfassen eine oder mehrere Elektrodenflächen am distalen Ende der Elektrode und einen Anschluss-Stecker am proximalen Ende der Elektrode. Letzterer dient als Schnittstelle zum implantierbaren Stimulationsgerät.

Wenn dem Patienten eine implantierbare Elektrode eingesetzt wird, ist es in der Regel empfehlenswert, einige vorläufige Tests durchzuführen, bevor die Elektrode an ihrem endgültigen Platz verankert und der Anschluss-Stecker der Elektrode mit dem implantierten Stimulationsgerät verbunden wird. Zu diesem Zweck wird der Anschluss-Stecker der Elektrode zeitweise mit einer Messeinrichtung, wie beispielsweise einem Patienten-Systemanalysegerät, verbunden, so dass eine Reihe von Stimulationsimpulsen unterschiedlicher Energien oder andere Testsignale der vorläufig eingesetzten Elektrode zugeführt werden können.

Der Anschluss dieser Messeinrichtung an den Anschluss-Stecker der Elektrode erfolgt mit Testanschluss-Kontakten beispielsweise in Form von Krokodilklemmen.

Die Problematik der Verbindung solcher Testanschluss-Kontakte mit dem Anschluss-Stecker der Elektrode soll im Folgenden anhand des Beispiels von Krokodilklemmen kurz beleuchtet werden. So ist die direkte Anklipsung von Krokodilklemmen an den Anschluss-Stecker zum einen bereits generell wenig zuverlässig, da die Krokodilklemmen während des Testbetriebes sich vom Anschluss-Stecker lösen können. Darüber hinaus kann bei einer schlechten Positionierung der Krokodilklemmen an den Anschluss-Kontakten des Anschluss-Steckers eine schlechte Kontaktverbindung vorliegen, die die Tests beeinflussen und zu falschen Rückschlüssen beispielsweise auf die Positionierung der Elektrode führen können. Auch eine Fehlkontaktierung und Kurzschlüsse können bei unachtsamer Anwendung solcher Krokodilklemmen auftreten.

Der Stand der Technik zeigt im Zusammenhang mit dieser Problematik einige Lösungsansätze. So offenbart die US 7,777,140 B2 eine Art Kontaktzange, in deren Zangenschenkel jeweils die einzelnen Kontaktelemente für die Anschluss-Kontakte des Elektroden-Anschluss-Steckers gelagert sind. Da der Anschluss-Stecker zwischen die Zangenschenkel eingelegt wird, ist allerdings kein vollständiger Berührungsschutz für die Anschluss-Kontakte des Elektrodensteckers gegeben. Auch kann ein falsches Einsetzen des Anschluss-Steckers in die Kontaktzange drohen, so dass insgesamt die Verbindungssicherheit dieser bekannten Adapterlösung zu wünschen übrig lässt.

Andere Adaptionslösungen zeigen die US 5,354,326 A und die US 7,753,696 B2. Allen diesen Lösungen ist gemein, dass eine passgenaue Ausrichtung der Testanschluss-Kontakte zu den Anschluss-Kontakten des Steckers der Elektrode erschwert ist. Auch ist insbesondere bei den in den vorgenannten Druckschriften gezeigten Adapterlösungen ein oftmals von Anwendern solcher Elektroden geforderter Mandrinwechsel bei in den Adapter eingelegter Position des Anschluss-Steckers während der Implantationsprozedur nicht möglich.

Das Dokument US 2002/0095079 A1 offenbart einen Adapter gemäß des Oberbegriffs des Anspruchs 1.

Ausgehend von den geschilderten Problemen des Standes der Technik liegt der Erfindung die Aufgabe zugrunde, einen Adapter zum mechanischen und elektrischen Anschluss einer implantierbaren Elektrode an eine Messeinrichtung zu schaffen, der eine einfache und sichere Adapterhandhabung, insbesondere eine verbesserte Positionierung der Anschluss-Kontakte der Elektrode zu den Testanschluss-Kontakten der Messeinrichtung zueinander gewährleistet.

Diese Aufgabe wird durch einen Adapter gelöst, der laut Patentanspruch 1 folgende Merkmale umfasst:
- ein Adaptergehäuse aus einem elektrisch isolierenden Werkstoff,
- eine an die Form des Anschluss-Steckers angepasste Aufnahme für den Anschluss-Stecker,
- mindestens ein im Adaptergehäuse angeordnetes Kontaktelement zur Kontaktverbindung zwischen dem mindestens einen Anschluss-Kontakt des Anschluss-Steckers und dem mindestens einen Testanschluss,
- eine am Adaptergehäuse zwischen einer Offen-Position und einer geschlossenen Kontakt-Position lageveränderlich gelagerten Aufnahme-Lade, in der die Aufnahme für den Anschluss-Stecker angeordnet ist, wobei
- in der Offen-Position der Anschluss-Stecker in die Aufnahme in einer definierten Position einsetzbar und wieder daraus entnehmbar ist, und
- durch das Überführen der Aufnahme-Lade in die Kontaktposition der mindestens eine Anschluss-Kontakt des Anschluss-Steckers mit dem Kontaktelement im Adaptergehäuse definiert in Kontakt bringbar ist.

Der Gegenstand des Patentanspruchs zeichnet sich dadurch aus, dass die Aufnahme-Lade eine zwischen der Offen- und der Kontakt-Position am Adaptergehäuse verschiebbare Schublade ist.

Deren Verschieberichtung kann vorzugsweise quer zur Längsrichtung des Anschluss-Steckers verlaufen.

Durch das Einsetzen des Anschluss-Steckers in eine definiert im Adaptergehäuse zwischen der Offen- und Kontakt-Position lagerveränderlich gelagerten verschiebbaren Schubladedient letztere als Positionierungshilfe für den Anschluss-Stecker, der so die Voraussetzung erfüllt, in der Kontakt-Position der verschiebbaren Schubladekorrekt mit den Testanschluss-Kontakten in Verbindung gebracht zu werden. Die verschiebbare Schubladenimmt dabei den Anschluss-Stecker lagerichtig auf und führt dessen Anschluss-Kontakte den im Adaptergehäuse angeordneten Kontaktelementen definiert und genau zu, so dass ein sicherer elektrischer Kontakt zu der Messeinrichtung beispielsweise über Krokodilklemmen hergestellt wird. Da letztere nicht direkt auf die Anschluss-Kontakte des Anschluss-Steckers der Elektrode aufgesetzt werden, ist der Elektroden-Anschluss-Stecker wirkungsvoll vor Beschädigungen, wie Kratzern oder Deformationen, geschützt.

Gemäß einer nicht im Gegenstand der Patentansprüche umfassten Ausführung kann die Aufnahme-Lade auch als zwischen den beiden genannten Positionen verschwenkbare Klapplade ausgebildet sein, wobei die Verschwenkrichtung dann wiederum quer zur Längsrichtung des Anschluss-Steckers verlaufen kann.

Beide vorgenannten Weiterbildungen sind konstruktiv relativ einfach zu realisieren.

In einer weiteren bevorzugten Ausführungsform ist die verschiebbare Schublade in der Offen- und/oder Kontakt-Position relativ zum Adaptergehäuse durch eine Verrastung festlegbar. Bevorzugter Weise werden beide Positionen gerastert. Durch die Rastfunktion in der Offen-Stellung ist ein kontrolliertes Positionieren und Einlegen des Anschluss-Steckers in den Adapter ermöglicht. Die Verrastung der geschlossenen verschiebbaren Schublade in der Kontakt-Position verhindert das versehentliche Öffnen und Herausfallen der Elektrode aus dem Adapter.

Bedienungskomfort und Handhabungssicherheit des erfindungsgemäßen Adapters können weiter dadurch gesteigert werden, dass die Verrastung durch einen Auslöseknopf lösbar ist. Letzterer kann an das Adaptergehäuse über eine einstückig mit diesen beiden verbundene Federzunge angebunden sein. Die Auslöseknopf-Konstruktion ist damit über einfache spritzgusstechnisch herzustellende Formmerkmale des Adaptergehäuses zu realisieren.

Gemäß einer weiteren bevorzugten Ausführungsform sind die Anschluss-Kontakte des Anschluss-Steckers in der Kontaktposition der verschiebbaren Schublade nach außen hin vollständig abdeckbar. Damit ist ein wirkungsvoller Berührungsschutz bei der Handhabung der Elektrode in Verbindung mit dem erfindungsgemäßen Adapter gegeben.

Weiterhin können die verschiebbare Schublade und das Adaptergehäuse stirnseitig vor dem in der verschiebbaren Schubladepositionierbaren Anschluss-Stecker mit einer Öffnung zum freien Zugriff auf ein durch den Anschluss-Stecker geführtes Zentrallumen der Elektrode sowohl in der Offen- als auch in der Kontakt-Position versehen sein. Diese Weiterbildung der Erfindung ermöglicht einen einfachen Wechsel eines in das Zentrallumen der Elektrode eingeführten Mandrins, ohne Adapter und Stecker trennen zu müssen. Dieser Vorteil fehlt den Kontaktierungsarten der Adapter, wie sie im eingangs genannten Stand der Technik gezeigt sind.

Weitere bevorzugte Ausbildungen beziehen sich auf das Kontaktelement im Adaptergehäuse, das eine nach außen hin offen liegende Kontaktfläche für jeden Testanschluss-Kontakt aufweisen kann. Vorzugsweise wird jede Kontaktfläche von einer entsprechenden Führung für den Testanschluss-Kontakt flankiert. Beispielsweise können so Krokodilklemmen sicher auf den Adapter aufgesetzt und mit dessen Kontaktelement elektrisch in Verbindung gebracht werden.

In der Regel weisen Anschluss-Stecker von implantierbaren Elektroden mehrere Anschluss-Kontakte auf. Insoweit kann der erfindungsgemäße Adapter für einen solchen Anschluss-Stecker zwei Kontaktelemente aufweisen, die jeweils einerseits mit einem Anschluss-Kontakt des Anschluss-Steckers in der verschiebbaren Schublade und andererseits mit jeweils einem Testanschluss-Kontakt einer Messeinrichtung in Verbindung zu bringen sind.

Zur sicheren Handhabung des Adapters und der daran anzuschließenden Messeinrichtung kann dann den offen liegenden Kontaktflächen der Kontaktelemente für die Testanschluss-Kontakte jeweils ein Polaritätssymbol zugeordnet sein.

Gemäß einer weiteren bevorzugten Ausführungsform weist das Adaptergehäuse zwei oder vier Kontaktelemente etwa zur Anwendung bei Schrittmacher- oder Defibrillator-Elektroden auf. Für andere Anwendungen sind auch acht und mehr Kontaktelemente denkbar.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1 und 2: perspektivische Ansichten schräg von oben eines Adapters mit Anschluss-Stecker in Offen- und Kontakt-Position der Adapter-Schublade mit Auslöseknopf,
- Fig. 3 und 4: perspektivische Ansichten analog Fig. 1 und 2 schräg von unten,
- Fig. 5: eine perspektivische Ansicht des Adapters gemäß Fig. 2 mit angeschlossenen Krokodilklemmen und gestecktem Mandrin,
- Fig. 6 und 7: eine perspektivische Detailansicht einer in den Adapter einzusetzenden Leiterplatte und eine weggeschnittene Perspektivansicht des Adapters,
- Fig. 8 und 9: perspektivische Ansichten schräg von oben eines Adapters in einer zweiten Ausführungsform mit Anschluss-Stecker in Offen- und Kontakt-Position der Adapter-Klapplade, welche nicht im Gegenstand des Patentanspruchs 1 umfasst ist
- Fig. 10 und 11: perspektivische Ansichten schräg von unten analog Fig. 8 und 9,
- Fig. 12: eine perspektivische Ansicht des Adapters gemäß Fig. 9 mit angeschlossenen Krokodilklemmen und gestecktem Mandrin,
- Fig. 13 und 14: perspektivische Ansichten schräg von oben eines Adapters in einer weiteren Ausführungsform ohne Auslöseknopf mit Anschluss-Stecker in Offen- und Kontakt-Position der Adapter-Schublade, sowie
- Fig. 15: eine perspektivische Ansicht eines Adapters in einer weiteren Ausführungsform mit vier Kontaktelementen.

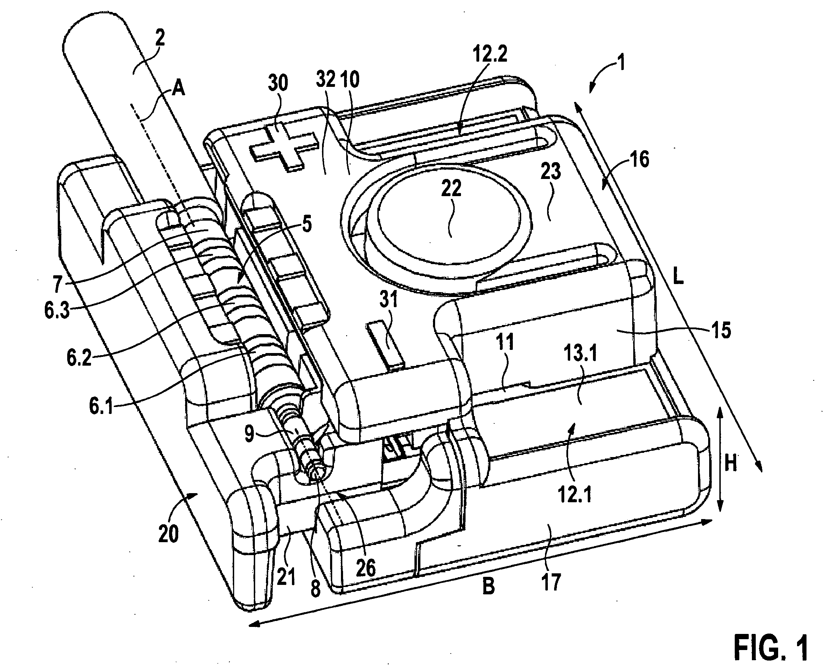

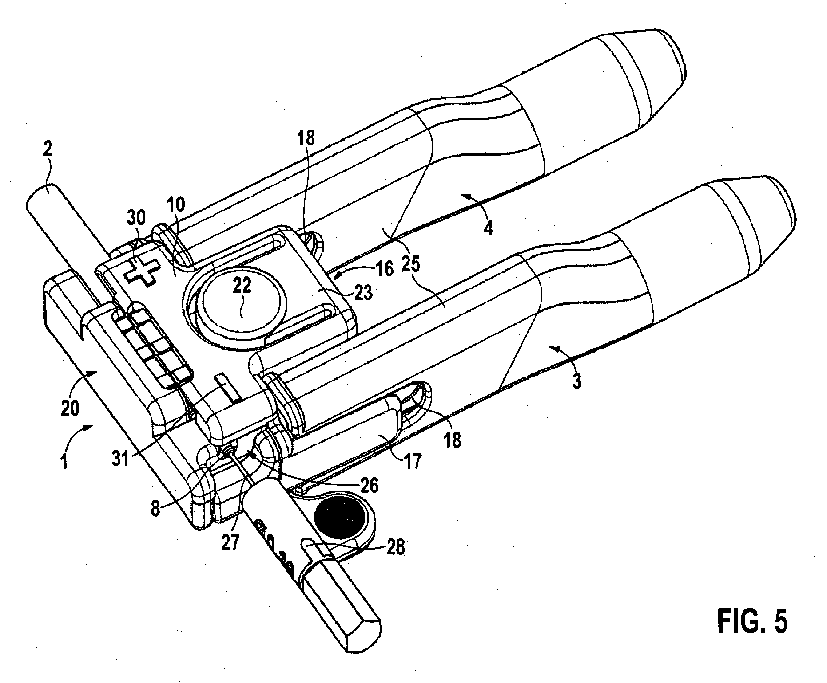

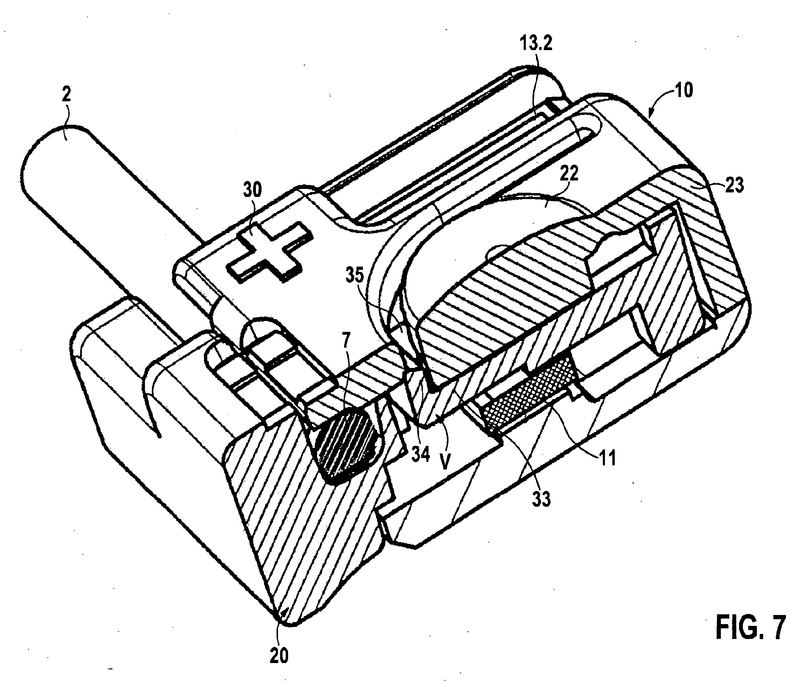

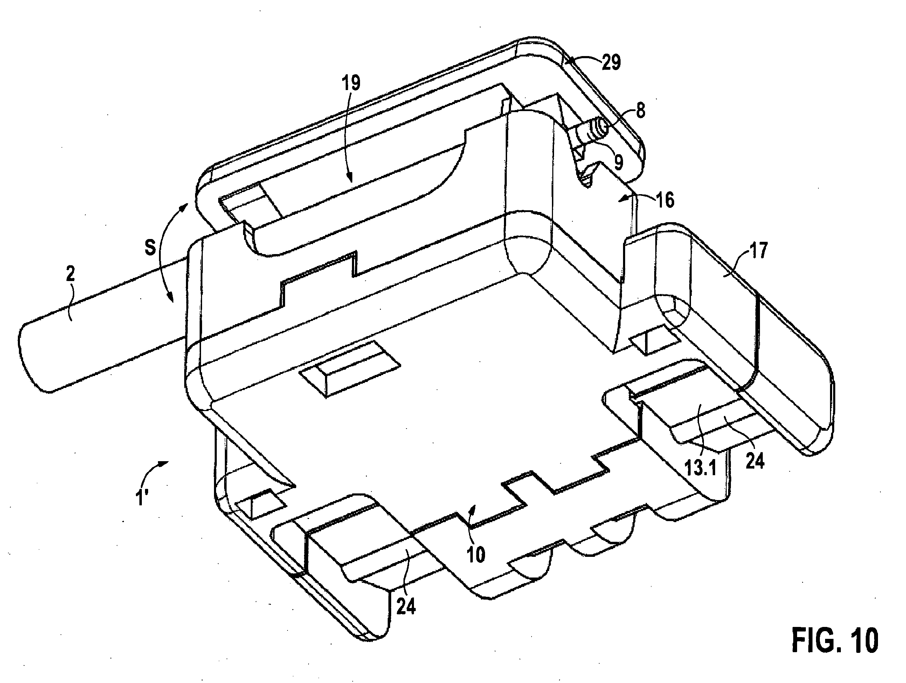

Die Fig. 1 bis 7 zeigen eine erste Ausführungsform eines Adapters 1 zum mechanischen und elektrischen Anschluss einer in den Zeichnungen weggeschnitten dargestellten, implantierbaren Elektrode 2 an eine nicht näher dargestellten Messeinrichtung mit Hilfe von Testanschluss-Kontakten, wie sie in Fig. 5 und 10 in Form von Krokodilklemmen 3, 4 beispielhaft gezeigt sind. Die Elektrode 2 weist dabei an ihrem insbesondere in Fig. 1, 8 und 13 erkennbaren proximalen Ende einen Anschluss-Stecker 5 auf, der vier Anschlusskontakte 6.1, 6.2, 6.3 in Form von Ringflächen am Steckerkörper 7 sowie einen Kontakt 6.4 an einem Pin 9 an der Steckerspitze aufweist. Ferner umfasst die Elektrode 2 ein zentrales Lumen 8, das am Ende der Elektrode 2 über den koaxial zur Elektrode 2 verlaufenden Pin 9 herausgeführt ist.

Das Adaptergehäuse 10 weist eine gegliederte Außenform auf, die im groben Umriss als quaderförmig mit einer Länge L, Breite B und Höhe H beschrieben werden kann. Es besteht aus einem elektrisch isolierenden Werkstoff, wie beispielsweise einem spritzgegossenen Kunststoffmaterial.

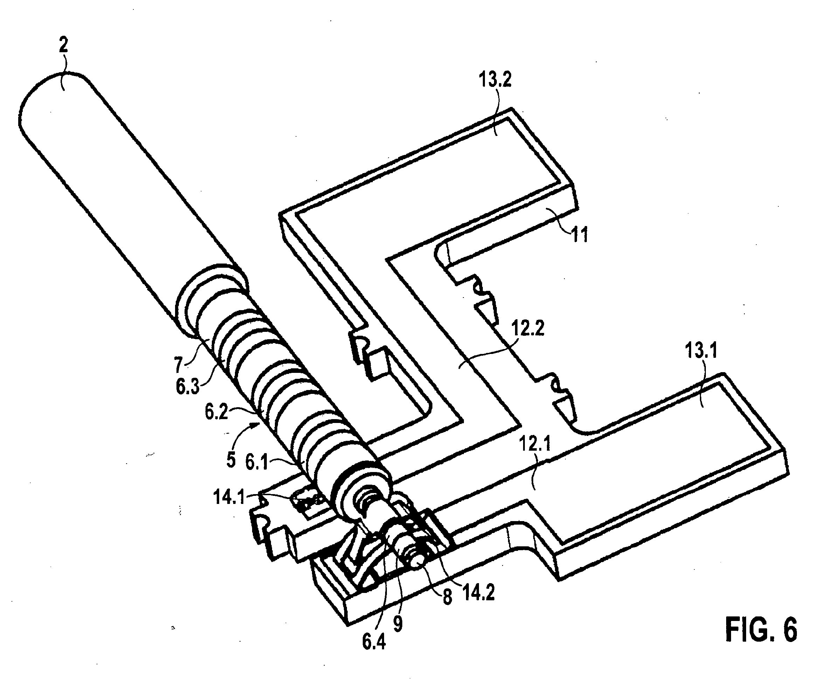

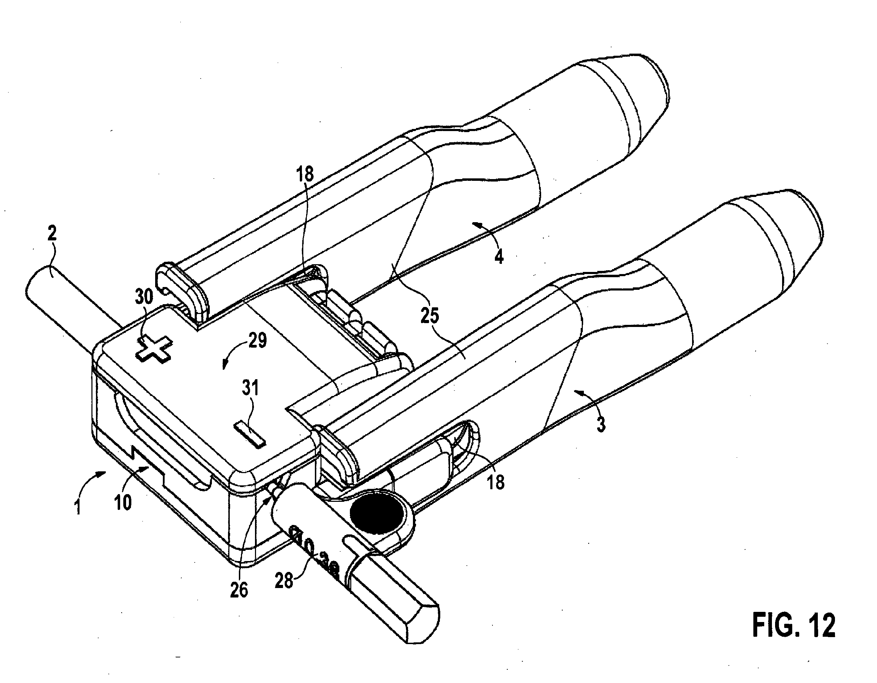

Im Adaptergehäuse 10 ist eine Leiterplatte 11 mit zwei Kontaktelementen 12.1, 12.2 in Form von metallischen Kontaktstreifen eingesetzt, von denen in den Fig. 1 bis 4 und 6 die für den Anschluss der Krokodilklemmen 3, 4 dienenden, offen liegende Kontaktflächen 13.1, 13.2 erkennbar sind. Im Verbindungsbereich zum Anschluss-Stecker 5 sind an den Kontaktelementen 12.1, 12.2 jeweils an der Leiterplatte 11 fixierte Federkontakte 14.1, 14.2 elektrisch angeschlossen, die den Kontakt zu den Anschluss-Kontakten 6.3 und 6.4 der Elektrode 2 bei geschlossenem Adapter 1 herstellen (siehe Fig. 6). Die vom Adaptergehäuse 10 durch die Seitenwangen 15 des Aufbaus 16 und die überstehenden Schmalseiten 17 gebildeten Führungen nehmen das "Maul" 18 der Krokodilklemmen 3, 4 auf (siehe Fig. 5 und 12), wobei die Klemmsicherheit noch durch die Stege 24 an der Unterseite des Adaptergehäuses 10 erhöht wird.

Für den Anschluss-Stecker 5 ist im Adapter 1 eine Aufnahme 19 vorgesehen, die bei der in Fig. 1 bis 7 gezeigten Ausführungsform in einer Schublade 20 angeordnet ist. Diese Aufnahme 19 entspricht der Form des Anschluss-Steckers 5, so dass dieser lagerichtig und exakt definiert in der Schublade 20 aufgenommen wird, wie dies Fig. 1 zeigt.

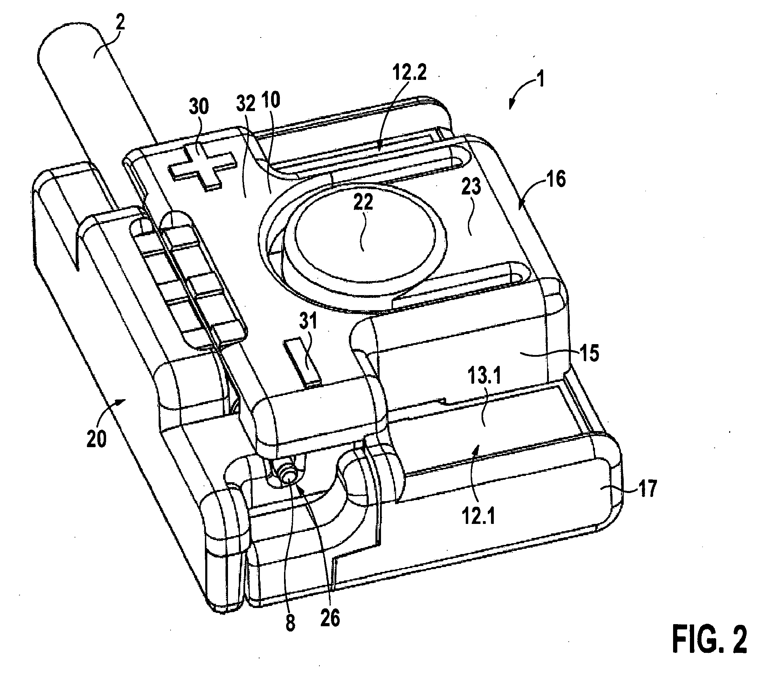

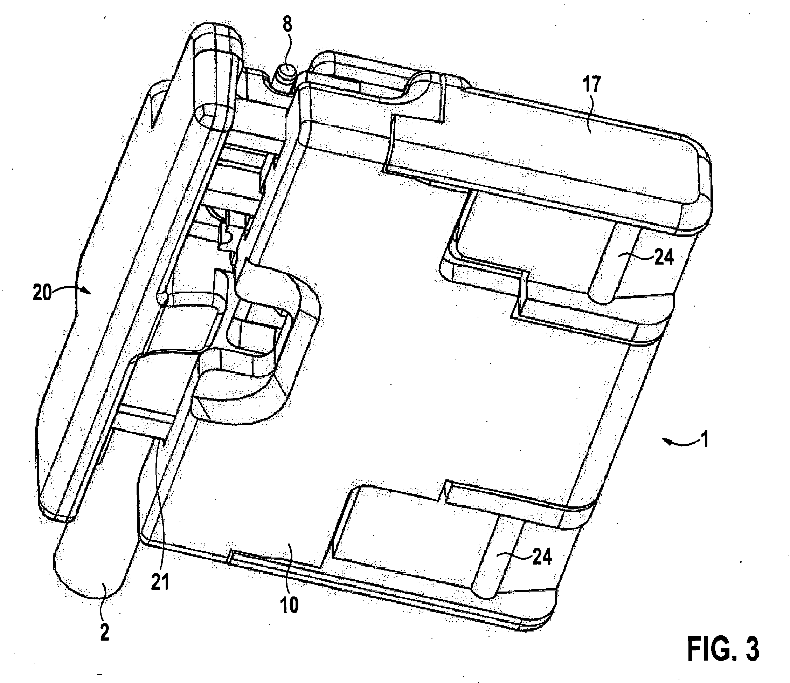

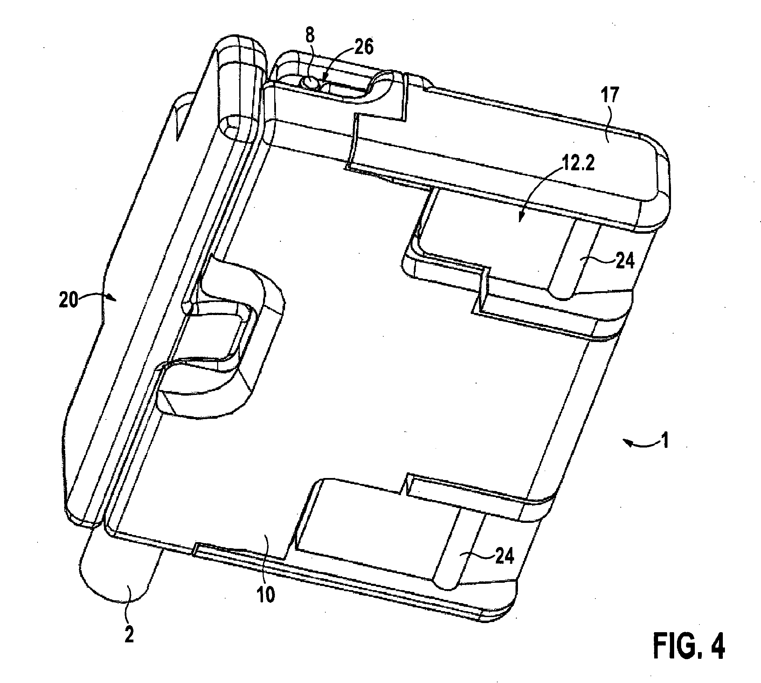

Die Schublade 20 ist in Richtung der Breite B des Adaptergehäuses 10 zwischen der in Fig. 1 und 3 gezeigten Offen-Position und der in Fig. 2, 4 und 5 gezeigten Kontakt-Position lageveränderlich, nämlich über entsprechende Führungen 21 verschiebbar am Adaptergehäuse 10 gelagert. In der Offen-Position gemäß Fig. 1 und 3, in der der Anschluss-Stecker 5 in die Aufnahme 19 einsetzbar ist, und in der in Fig. 2 und 4 gezeigten Kontakt-Position ist die Schublade 20 relativ zum Adaptergehäuse 10 verrastet. Die Verrastung V ist durch eine an der Schublade 20 ausgebildete Rastzunge 33 gebildet, die mit einem Vorsprung 34 in eine Rastausnehmung 35 im Adaptergehäuse 10 vor einem in Fig. 1, 2, 5 und 7 erkennbaren Auslöseknopf 22 eingreift, der über eine elastische Federzunge 23 an das Adaptergehäuse 10 in Richtung der Höhe H verlagerbar angebunden ist. Die Verrastung V ist durch den Auslöseknopf 22 aufhebbar, indem dieser gedrückt und der Vorsprung 34 damit aus der Rastausnehmung 35 gehoben wird. Die Schublade 20 wird damit frei beweglich.

Aus der in Fig. 1 und 3 gezeigten Offen-Position wird die Schublade 20 mit dem eingelegten Anschluss-Stecker 5 quer zu dessen Längsrichtung A in das Adaptergehäuse 10 eingeschoben. In der in Fig. 2, 4 und 5 gezeigten Kontakt-Position der Schublade 20 sind dann die beiden Anschlusskontakte 6.3 und 6.4 über die Federkontakte 14.1, 14.2 der Kontaktelemente 12.1, 12.2 elektrisch herausgeführt. Durch die Verrastung der Schublade 20 in dieser Kontakt-Position ist eine sichere Verbindung zwischen dem Anschluss-Stecker 5 und den Kontaktelementen 12.1, 12.2 gegeben.

Auf diese wird, wie Fig. 5 zeigt, jeweils eine Krokodilklemme 3, 4 aufgeschoben. Deren Sitz auf den Kontaktelementen 12.1, 12.2 wird dabei noch durch die in Fig. 3 und 4 dargestellten stegförmigen Ausprägungen 24 an der Unterseite des Adaptergehäuses 10 verbessert. Diese Stege sollen das Abrutschen der Krokodilklemmen 3, 4 verhindern. Da die Krokodilklemmen 3, 4 nach außen hin mit ihren Kontakten durch Isolierschuhe 25 abgedeckt sind, ist insgesamt eine saubere, vor Berührungen geschützte Kontaktverbindung zwischen einer Messeinrichtung über die Krokodilklemmen 3, 4 und den Adapter 1 zum Anschluss-Stecker 5 realisiert.

Wie ferner in Fig. 5 dargestellt ist, weist die Schublade 20 und das Adaptergehäuse 10 eine Öffnung 26 auf, über die der Pin 9 mit dem Lumen 8 der Elektrode 2 auch in der in Fig. 2, 4 und 5 gezeigten Kontakt-Position frei zugänglich ist. Somit kann der in Fig. 5 eingezeichnete Mandrin 27 durch Ziehen an seinem Handgriff 28 einfach ausgetauscht oder anderweitig betätigt werden, ohne dass sich an der Positionierung des Anschluss-Steckers und Adapters 1 etwas ändern müsste.

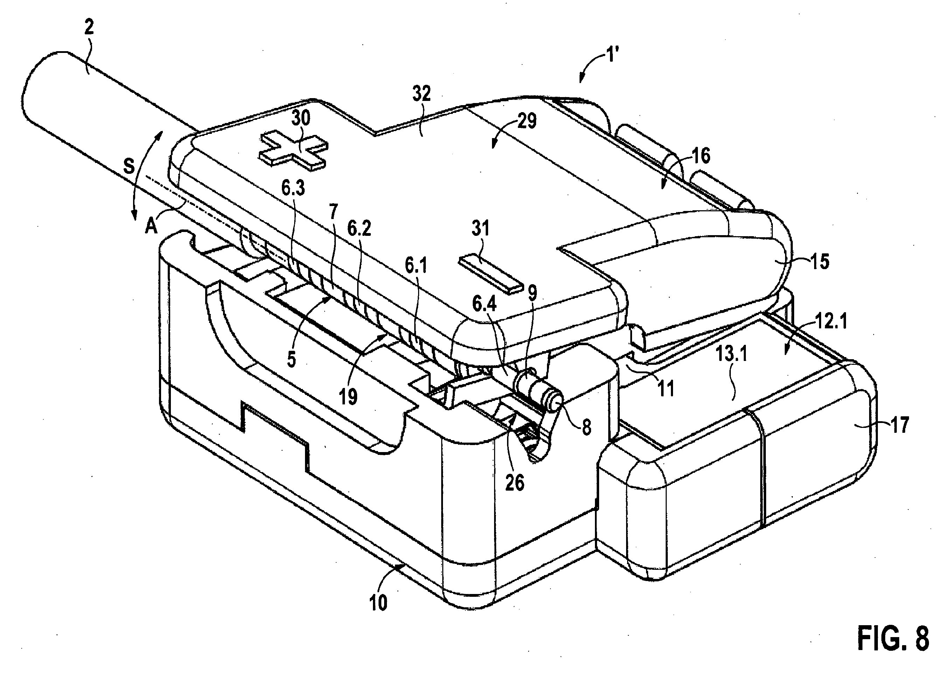

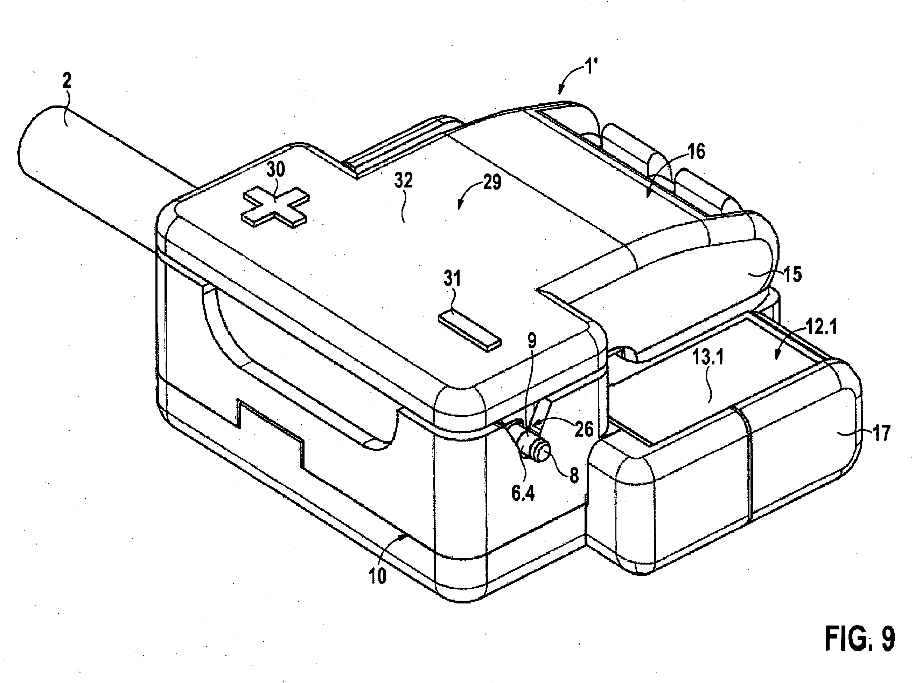

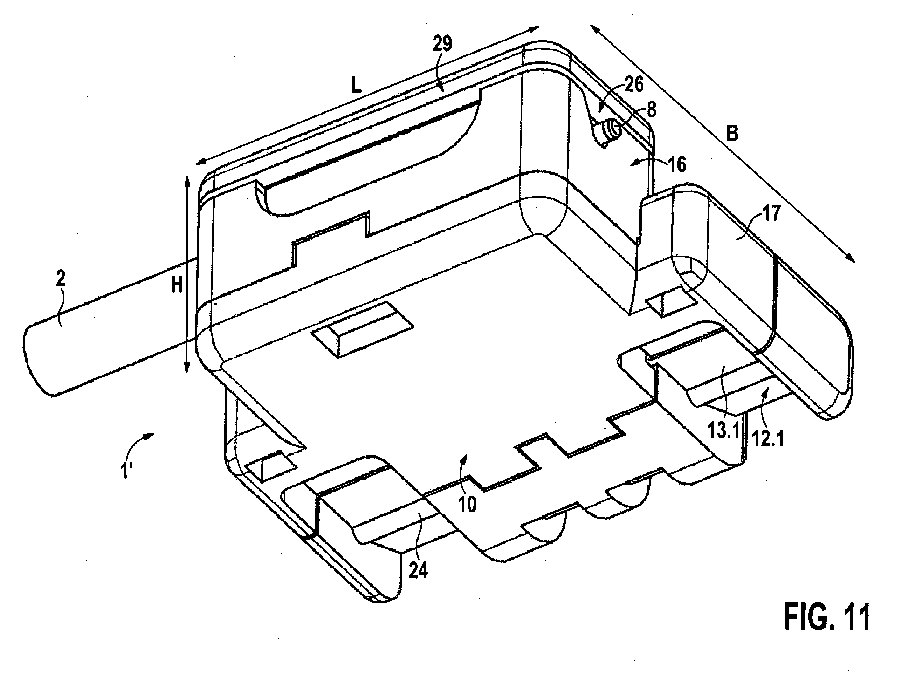

In den Fig. 8 bis 12 ist eine zweite Ausführungsform des Adapters 1' gezeigt, welche nicht im Gegenstand des Patentanspruchs 1 umfasst ist. Diese unterscheidet sich von der Ausführungsform gemäß Fig. 1 bis 7 in der Ausführung der Aufnahme-Lade. Diese ist hier durch eine Klapplade 29 realisiert, die am Adaptergehäuse 10 um eine parallel zur Länge verlaufende Schwenkachse S schwenkbar gelagert ist. In der in Fig. 8 und 10 gezeigten Offen-Position ist die Klapplade 29 hochgeklappt, so dass die Aufnahme 19 von der Seite her für den Anschluss-Stecker 5 der Elektrode 2 zugänglich ist (siehe Fig. 8 und 10).

Durch die Schwenkbewegung um die Schwenkachse S wird der in die Aufnahme 19 eingelegte Anschluss-Stecker 5 in das Adaptergehäuse 10 hineingeschoben und dort intern mit den Kontaktelementen 12.1, 12.2 mechanisch und elektrisch verbunden. Letztere sind analog dem Ausführungsbeispiel gemäß Fig. 1 bis 7 aufgebaut und bedürfen daher keiner nochmaligen Erörterung. Alle mit dem Adapter 1 gemäß Fig. 1 bis 7 übereinstimmenden Komponenten des Adapters 1' sind mit identischen Bezugszeichen versehen, so dass auf die Beschreibung gemäß Fig. 1 bis 7 verwiesen werden kann. Zu ergänzen ist, dass die Klapplade 29 in der Offen- und Kontakt-Position gemäß Fig. 8 und 10 bzw. Fig. 9, 11 und 12 ebenso verrastbar ist. Die Lösung der Verrastung erfolgt durch einfachen Druck oder Zug an der Klapplade 29. Ein Auslöseknopf ist hier nicht vorgesehen.

Wichtig ist zu erwähnen, dass - wie Fig. 12 zeigt - auch der Adapter 1' so ausgelegt ist, dass über eine Öffnung 26 ein Mandrin 27 über seinen Handgriff 28 während des Anschlusses einer Messeinrichtung durch die Krokodilklemmen 3, 4 in geeigneter Weise manövriert werden kann.

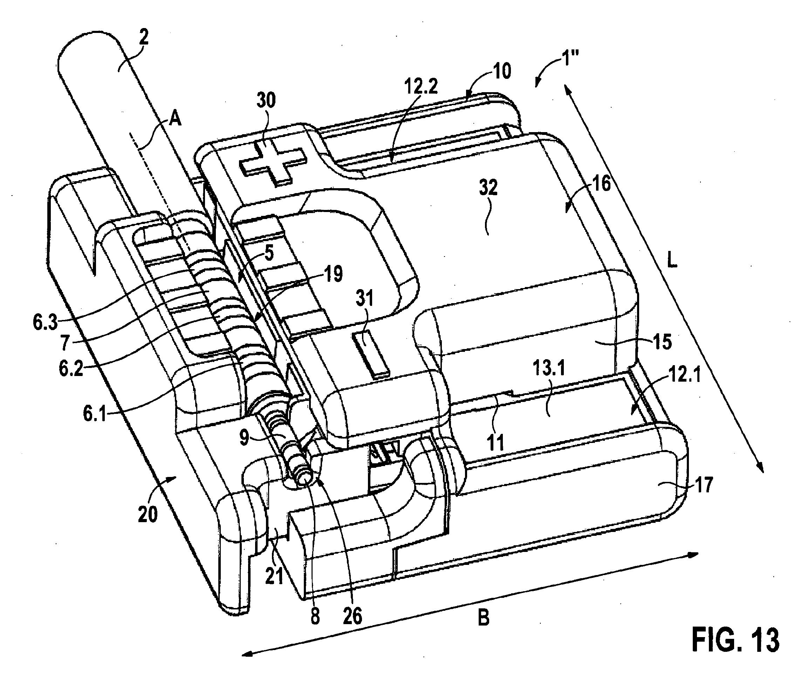

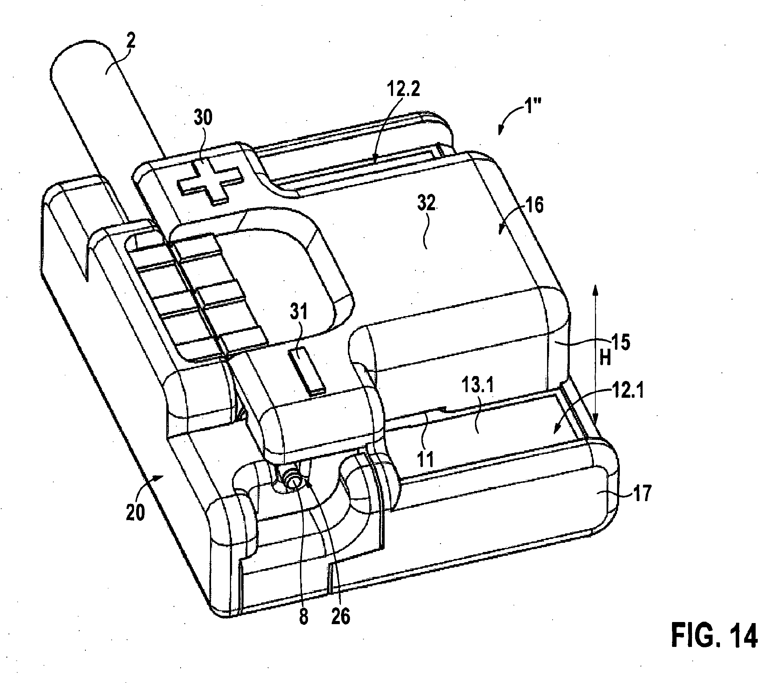

In Fig. 13 und 14 ist eine weitere Ausführungsform eines Adapters 1 " gezeigt, die der Ausführungsform gemäß Fig. 1 bis 7 entspricht. Einziger Unterschied ist, dass der Auslöseknopf 22 für die Aufhebung der Verrastung der Schublade 20 in der Offen- und Kontakt-Position weggelassen ist. Diese Verrastungen können - analog dem Ausführungsbeispiel gemäß den Fig. 8 bis 12 - durch einfachen Zug oder Druck an der Schublade 20 überwunden werden.

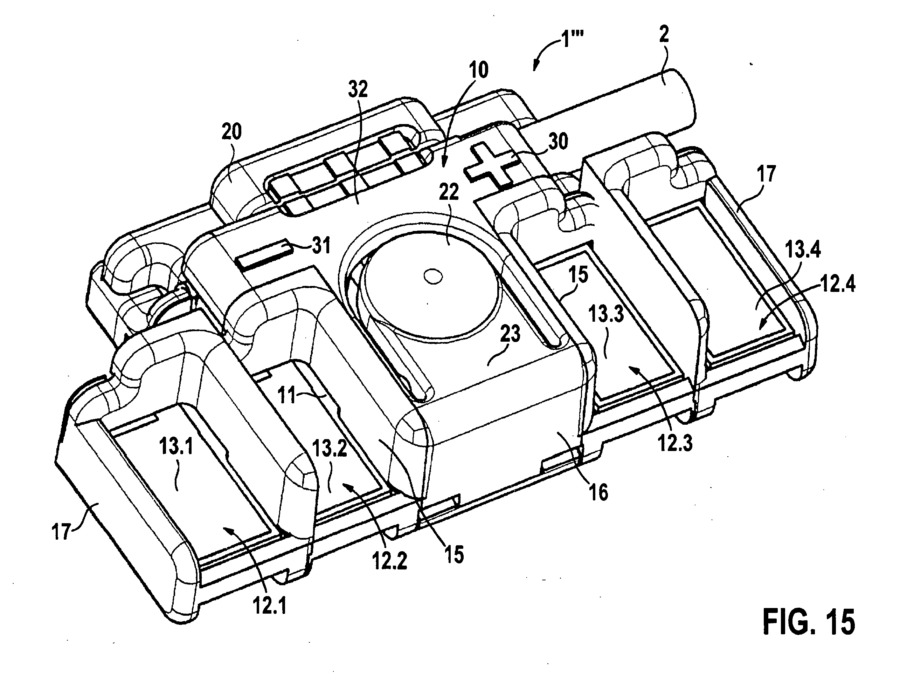

In Fig. 15 ist schließlich ein Adapter 1 "' gezeigt, der in der grundsätzlichen Ausgestaltung dem Adapter 1 gemäß Fig. 1 bis 7 entspricht, allerdings zum Kontaktieren aller vier Anschlusskontakte 6.1 bis 6.4 des Anschluss-Steckers 5 ausgelegt ist. Es sind mit dem Adapter 1 übereinstimmende Komponenten mit identischen Bezugszeichen versehen und bedürfen keiner nochmaligen Erläuterung. Im Unterschied zum vorgenannten Ausführungsbeispiel zeigt die Version des Adapter 1"' gemäß Fig. 15 allerdings vier Kontaktelemente 12.1 bis 12.4 auf der Leiterplatte 11 mit entsprechend offen liegenden Kontaktflächen 13.1 bis 13.4, an denen jeweils Krokodilklemmen angesetzt werden können. Die Kontaktelemente 12.1 bis 12.4 auf der Leiterplatte 11 sind im Inneren des Adaptergehäuses 10 wiederum analog Fig. 6 an Federkontakten angeschlossen, die mit den einzelnen Anschlusskontakten 6.1 bis 6.4 in der in Fig. 15 gezeigten geschlossenen Position des Adapters 1"' elektrisch in Verbindung gebracht werden können.

Allen vier Ausführungsbeispielen des Adapters 1, 1', 1", 1"' ist schließlich gemein, dass das Adaptergehäuse 10 mit Polaritätssymbolen 30, 31 " + " und " - " versehen ist, die den jeweiligen Kontaktflächen 13.1, 13.2 zugeordnet sind. Die Polaritätssymbole 30, 31 sind erhaben auf der Oberseite 32 des Aufbaus 16 der Adaptergehäuse 10 ausgebildet.

## Patentansprüche

1. Adapter zum mechanischen und elektrischen Anschluss einer implantierbaren Elektrode (2) mit ihrem proximalen, mindestens einen Anschluss-Kontakt (6) für eine Elektrodenfläche der Elektrode (2) aufweisenden Anschluss-Stecker (5) an mindestens einen Testanschluss-Kontakt, insbesondere an eine Krokodilklemme (3, 4), einer Messeinrichtung, umfassend
- ein Adaptergehäuse (10) aus einem elektrisch isolierenden Werkstoff,
- eine an die Form des Anschluss-Steckers (5) angepasste Aufnahme (19) für den Anschluss-Stecker (5),
- mindestens ein im Adaptergehäuse (10) angeordnetes Kontaktelement (12) zur Kontaktverbindung zwischen dem mindestens einen Anschluss-Kontakt (6) des Anschluss-Steckers (5) und dem mindestens einen Testanschluss (3, 4),
- eine am Adaptergehäuse (10) zwischen einer Offen-Position und einer geschlossenen Kontakt-Position lageveränderlich gelagerten Aufnahme-Lade (20, 29), in der die Aufnahme (19) für den Anschluss-Stecker (5) angeordnet ist, wobei
- in der Offen-Position der Anschluss-Stecker (5) in die Aufnahme (19) in einer definierten Position einsetzbar und wieder daraus entnehmbar ist, und
- **durch** das Überführen der Aufnahme-Lade (20, 29) in die Kontaktposition der mindestens eine Anschluss-Kontakt (6) des Anschluss-Steckers (5) mit dem Kontaktelement (12) im Adaptergehäuse (10) definiert in Kontakt bringbar ist,
**dadurch gekennzeichnet, dass**
die Aufnahme-Lade eine zwischen der Offen- und der Kontakt-Position am Adaptergehäuse (10) verschiebbare Schublade (20) ist.

2. Adapter nach Anspruch 1, **dadurch gekennzeichnet, dass** die Verschieberichtung der Schublade (20) quer zur Längsrichtung (A) des Anschluss-Steckers (5) verläuft.

3. Adapter nach einem der vorgenannten Ansprüche, **dadurch gekennzeichnet, dass** die verschiebbare Schublade (20) in der Offen- und/oder Kontakt-Position relativ zum Adaptergehäuse (10) durch eine Verrastung (V) festlegbar ist.

4. Adapter nach Anspruch 3, **dadurch gekennzeichnet, dass** die Verrastung (V) durch einen Auslöseknopf (22) lösbar ist.

5. Adapter nach Anspruch 4, **dadurch gekennzeichnet, dass** der Auslöseknopf (22) an das Adaptergehäuse (10) über eine einstückig mit beiden verbundene Federzunge (23) angebunden ist.

6. Adapter nach einem der vorgenannten Ansprüche, **dadurch gekennzeichnet, dass** der mindestens eine Anschluss-Kontakt (6) des Anschluss-Steckers (5) in der Kontaktposition der verschiebbaren Schublade (20) nach außen hin vollständig abdeckbar ist.

7. Adapter nach einem der vorgenannten Ansprüche, **dadurch gekennzeichnet, dass** die verschiebbare Schublade (20) und das Adaptergehäuse (10) stirnseitig vor dem in der verschiebbaren Schublade (20) positionierbaren Anschluss-Stecker (5) mit einer Öffnung (26) zum freien Zugriff sowohl in der Offen- als auch Kontakt-Position auf ein durch den Anschluss-Stecker (5) geführtes Zentrallumen (8) der Elektrode (2) versehen sind.

8. Adapter nach einem der vorgenannten Ansprüche, **dadurch gekennzeichnet, dass** das mindestens eine Kontaktelement (12) im Adaptergehäuse (10) eine nach außen hin offen liegende Kontaktfläche (13) für den Testanschluss-Kontakt (3, 4) aufweist.

9. Adapter nach Anspruch 8, **dadurch gekennzeichnet, dass** die Kontaktfläche (13) von einer Führung (21) für den Testanschluss-Kontakt (3, 4) flankiert ist.

10. Adapter nach einem der vorgenannten Ansprüche, **dadurch gekennzeichnet, dass** bei einem Adapter für Anschluss-Stecker (5) mit mehreren Anschluss-Kontakten (6.1, 6.2, 6.3, 6.4) die verschiebbare Schublade (20) und das Adaptergehäuse (10) zur elektrischen Verbindung von zwei oder vier Anschluss-Kontakten (6.1, 6.2, 6.3, 6.4) mit zwei Testanschluss-Kontakten (3, 4) über jeweils ein Kontaktelement (12.1, 12.2, 12.3, 12.4) ausgelegt sind.

11. Adapter nach Anspruch 10, **dadurch gekennzeichnet, dass** den Kontaktelementen (12.1, 12.2), insbesondere den offen liegenden Kontaktflächen (13.1, 13.2) der Kontaktelemente (12.1, 12.2) jeweils ein Polaritätssymbol (30, 31) zugeordnet ist.

12. Adapter nach einem der vorgenannten Ansprüche, **dadurch gekennzeichnet, dass** das Adaptergehäuse (10) mehr als vier, vorzugsweise acht Kontaktelemente zum Anschluss von Testanschluss-Kontakten aufweist.

## Claims

1. An adapter for mechanically and electrically connecting an implantable electrode (2), via the proximal connector plug (5) thereof, to at least one test terminal contact, in particular to an alligator clip (3, 4), of a measuring unit, wherein said connector plug has at least one terminal contact (6) for an electrode surface of the electrode (2), comprising
- an adapter housing (10) made of an electrically insulating material,
- a receptacle (19) for the connector plug (5), which is adapted to the shape of the connector plug (5),
- at least one contact element (12) disposed in the adapter housing (10) for establishing contact between the at least one terminal contact (6) of the connector plug (5) and the at least one test terminal (3, 4),
- a receiving tray (20, 29), which is mounted on the adapter housing (10) so as to be displaceable between an open position and a closed contact position, wherein the receptacle (19) for the connector plug (5) is disposed in said receiving tray, wherein
- in the open position, the connector plug (5) can be inserted, in a defined position, into the receptacle (19) and removed therefrom, and
- the at least one terminal contact (6) of the connector plug (5) can be brought into contact with the contact element (12) in the adapter housing (10) in a defined manner by transferring the receiving tray (20, 29) into the contact position,
**characterized in that**
the receiving tray is a drawer (20) that is displaceable on the adapter housing (10) between the open position and the contact position.

2. The adapter according to claim 1, **characterized in that** the displacement direction of the drawer (20) extends transversely to the longitudinal direction (A) of the connector plug (5).

3. The adapter according to any one of the aforementioned claims, **characterized in that** the displaceable drawer (20) can be fixed in the open position and/or the contact position relative to the adapter housing (10) by means of a snap-in connection (V).

4. The adapter according to claim 3, **characterized in that** the snap-in connection (V) can be released by means of a release button (22).

5. The adapter according to claim 4, **characterized in that** the release button (22) is joined to the adapter housing (10) via a spring tab (23) connected to both as one piece.

6. The adapter according to any one of the aforementioned claims, **characterized in that** the at least one terminal contact (6) of the connector plug (5) can be completely covered toward the outside in the contact position of the displaceable drawer (20).

7. The adapter according to any one of the aforementioned claims, **characterized in that** the displaceable drawer (20) and the adapter housing (10) are provided, on the end faces thereof, with an opening (26) in front of the connector plug (5), which can be positioned in the displaceable drawer (20), for free access - in both the open position and the contact position - to a central lumen (8) of the electrode (2), which extends through the connector plug (5).

8. The adapter according to any one of the aforementioned claims, **characterized in that** the at least one contact element (12) in the adapter housing (10) has a contact surface (13) for the test terminal contact (3, 4), which is exposed toward the outside.

9. The adapter according to claim 8, **characterized in that** the contact surface (13) is flanked by a guide (21) for the test terminal contact (3, 4).

10. The adapter according to any one of the aforementioned claims, **characterized in that**, in the case of an adapter for connector plugs (5) comprising a plurality of terminal contacts (6.1, 6.2, 6.3, 6.4), the displaceable drawer (20) and the adapter housing (10) are designed to electrically connect two or four terminal contacts (6.1, 6.2, 6.3, 6.4) to two test terminal contacts (3, 4) via one contact element (12.1, 12.2, 12.3, 12.4) in each case.

11. The adapter according to claim 10, **characterized in that** a polarity symbol (30, 31) is assigned to each of the contact elements (12.1, 12.2), in particular to the exposed contact surfaces (13.1, 13.2) of the contact elements (12.1, 12.2).

12. The adapter according to any one of the aforementioned claims, **characterized in that** the adapter housing (10) comprises more than four, preferably eight contact elements for connecting test terminal contacts.

## Revendications

1. Adaptateur pour le raccordement mécanique et électrique d'une électrode implantable (2) avec son connecteur (5) proximal comportant au moins un contact de raccordement (6) pour une surface d'électrode de l'électrode (2), à au moins un contact de raccordement d'essai, en particulier à une pince crocodile (3, 4) d'un dispositif de mesure, comprenant
- un boîtier d'adaptateur (10) constitué d'un matériau électriquement isolant,
- un logement (19) adapté à la forme du connecteur (5) pour le connecteur (5),
- au moins un élément de contact (12) agencé dans le boîtier d'adaptateur (10) pour la liaison par contact entre l'au moins un contact de raccordement (6) du connecteur (5) et l'au moins un contact d'essai (3, 4),
- un tiroir de logement (20, 29) monté sur le boîtier d'adaptateur (10) de façon déplaçable entre une position ouverte et une position de contact fermée, dans lequel est agencé le logement (19) pour le connecteur (5), dans lequel
- dans la position ouverte, le connecteur (5) peut être inséré dans le logement (19) dans une position définie, puis ressorti de celui-ci, et
- le déplacement du tiroir de logement (20, 29) dans la position de contact permet de mettre en contact l'au moins un contact de raccordement (6) du connecteur (5) de façon définie avec l'élément de contact (12) dans le boîtier d'adaptateur (10),
**caractérisé en ce que**
le tiroir de logement est un tiroir (20) coulissant sur le boîtier d'adaptateur (10) entre la position ouverte et la position de contact.

2. Adaptateur selon la revendication 1, **caractérisé en ce que** la direction de coulissement du tiroir (20) s'étend transversalement à la direction longitudinale (A) du connecteur (5).

3. Adaptateur selon l'une des revendications précédentes, **caractérisé en ce que** le tiroir coulissant (20) peut être fixé par un moyen d'encliquetage (V) dans la position ouverte et/ou dans la position de contact par rapport au boîtier d'adaptateur (10).

4. Adaptateur selon la revendication 3, **caractérisé en ce que** le moyen d'encliquetage (V) peut être défait par un bouton de libération (22).

5. Adaptateur selon la revendication 4, **caractérisé en ce que** le bouton de libération (22) est relié au boîtier d'adaptateur (10) par une languette élastique (23) reliée aux deux d'une seule pièce.

6. Adaptateur selon l'une des revendications précédentes, **caractérisé en ce que** l'au moins un contact de raccordement (6) du connecteur (5) peut être entièrement recouvert vers l'extérieur dans la position de contact du tiroir coulissant (20).

7. Adaptateur selon l'une des revendications précédentes, **caractérisé en ce que** le tiroir coulissant (20) et le boîtier d'adaptateur (10) sont pourvus, du côté frontal, en amont du connecteur (5) apte à être positionné dans le tiroir coulissant (20), d'une ouverture (26) permettant d'accéder librement à la lumière centrale (8) de l'électrode (2) traversant le connecteur (5), aussi bien dans la position ouverte que dans la position de contact.

8. Adaptateur selon l'une des revendications précédentes, **caractérisé en ce que** l'au moins un élément de contact (12) dans le boîtier d'adaptateur (10) comporte une surface de contact (13) exposée vers l'extérieur, pour le contact de raccordement d'essai (3, 4).

9. Adaptateur selon la revendication 8, **caractérisé en ce que** la surface de contact (13) est encadrée par un guidage (21) pour le contact de raccordement d'essai (3, 4).

10. Adaptateur selon l'une des revendications précédentes, **caractérisé en ce qu'**avec un adaptateur pour des connecteurs (5) à plusieurs contacts de raccordement (6.1, 6.2, 6.3, 6.4), le tiroir coulissant (20) et le boîtier d'adaptateur (10) sont conçus pour la liaison électrique de deux ou quatre contacts de raccordement (6.1, 6.2, 6.3, 6.4) avec deux contacts de raccordement d'essai (3, 4) respectivement par le biais d'un élément de contact (12.1, 12.2, 12.3, 12.4).

11. Adaptateur selon la revendication 10, **caractérisé en ce qu'**un symbole de polarité (30, 31) est respectivement attribué aux éléments de contact (12.1, 12.2), en particulier aux surfaces de contact exposées (13.1, 13.2) des éléments de contact (12.1, 12.2).

12. Adaptateur selon l'une des revendications précédentes, **caractérisé en ce que** le boîtier d'adaptateur (10) comporte plus de quatre éléments de contact, de préférence huit, pour le raccordement de contacts de raccordement d'essai.
